# EUROPÄISCHE PATENTSCHRIFT

(11) **EP 0 554 670 B1**
(45) Veröffentlichungstag und Bekanntmachung des Hinweises auf die Patenterteilung: **05.09.2001**
(21) Anmeldenummer: 93100339.6
(22) Anmeldetag: 12.01.1993
(51) Int. Cl.: C12N 15/13, C12N 5/20, C12N 15/62, C12P 21/08, A61K 39/395, C07K 16/28, G01N 33/577

(54) **Spezifische Antikörper gegen aktivierte Plättchen, ihre Herstellung und ihre Verwendung in Diagnose und Therapie**
Specific antibodies against activated platelets, their production and applications in diagnostics and therapeutics
Anticorps spécifiques contre les plaquettes activée, leur fabrication et applications diagnostiques et thérapeutiques

(30) Priorität: 07.02.1992 DE 4203545
(43) Veröffentlichungstag der Anmeldung: 11.08.1993
(73) Patentinhaber: Dade Behring Marburg GmbH, 35001 Marburg (DE)
(72) Erfinder: Bosslet, Klaus, W-3550 Marburg (DE); Kehrel, Beate, W-4400 Münster (DE); Seemann, Gerhard, W-3550 Marburg-Elnhausen (DE)

(56) Entgegenhaltungen:
- EP-A- 0 443 404
- EP-A- 0 514 721
- WO-A-91/06858
- WO-A-91/10424
- WO-A-92/17499
- US-A- 4 610 960
- NATURE. Bd. 349, Nr. 10 , LONDON GB Seiten 293 - 299 WINTER, G.; MILSTEIN, C.; 'Man-made antibodies'
- JOURNAL OF BIOLOGICAL CHEMISTRY. Bd. 266, Nr. 3 , 25. Januar 1991 , BALTIMORE US Seiten 1740 - 1745 ASCH AS;TEPLER J;SILBIGER S;NACHMAN RL; 'Cellular attachment to thrombospondin. Cooperative interactions between receptor systems.'

## Beschreibung

Die Hybridomatechnologie erlaubt es, gegen beliebige Epitope auf definierten oder undefinierten Antigenen selektive monoklonale Antikörper (MAK) herzustellen. Hierzu können als Immunogene hochgereinigte, definierte Antigene oder auch Antigenmischungen, Zellen oder Gewebe verwendet werden, wobei für das Auffinden hochselektiver MAK auch ein geeignetes Immunogen gefunden werden muß. Mit dieser Technologie ist es nun gelungen, MAK zu entwickeln, die in der Lage sind, nach i.v. Injektion z.B. an Tumoren, entzündliche Prozesse und Gefäßverschlüsse im menschlichen Körper zu binden. Diese präferentielle Bindung von MAK an die entsprechenden Zielstrukturen (Tumor, entzündlicher Prozeß, Gefäßverschluß) wird bereits im Rahmen der nuklearmedizinischen Diagnostik genutzt. Die nuklearmedizinische Spezialdisziplin, die sich mit der Injektion radioaktiv markierter MAK zum Nachweis von Zielstrukturen beschäftigt, ist die Immunszintigraphie (Mach,J.P., Buchegger, F., Forni, M., Ritschard, J., Berche, C., Lumbraso, J.-O., Schreyer, M., Girardet, C., Accolla, R.C., Carrel, S. (1981) Immunology today 2:239-249). Ihr stehen z.Z. keine MAK zur Verfügung, die selektiv an Plättchen binden und im wesentlichen nicht an anderes menschliches Normalgewebe. Selbst die MAK, die gegen α-Granula Proteine von Plättchen gerichtet sind, binden zwar präferentiell an aktivierte Plättchen, zeigen aber doch deutliche Bindung an Epitope auf anderen Geweben. So reagieren z.B. MAK, die gegen Thrombospondin (TSP), ein α-Granula Protein (Baenziger, N.L., Brodie, G.N., Majerus, P.W. (1971). Proc. Nat. Acad. Sci. USA 68:240-243; Ganguly, P. (1971). J. Biol. Chem. 246:4286-4290) gerichtet sind, mit Epitopen im Drüsenepithel der Haut und Lunge, den Verbindungsstücken zwischen Dermis und Epidermis (Wight, T.N., Raugi, G.J., Mumby, S.M., Bornstein, D. (1985). J. Histochem. Cytochem. 33:295-302), dem Knorpel (Miller, R.R., McDevitt, C.A. (1988). Biochem. Biophys. Res. Commun. 153:708-714), decalzifiziertem Knochen (Robey, P.G., Young, M.F., Fisher, L.W., McClain, T.D. (1989). J. Cell. Biol. 108:719-727), Nabelschnurarterien (Fauvel-Lafeve, F., Legrand, Y.J. (1988). Thromb. Res. 50:305-316), Typ II Pneumozyten (Sage, H., Farin, F.M., Striker, G.E., Fisher, A.B. (1983). Biochemistry 22:2148-2155), Megakaryozyten (Beckstead, J.H., Stenberg, P.E., McEver, R.P., Shuman, M.A., Bainton, D.F. (1986). Blood 67:285-293) sowie proliferierenden Endothelzellen, glatter Muskulatur und Fibroblasten (Mumby, S.M., Abbott-Brown, D., Raugi, G.J., Bornstein, P. (1984). J. Cell. Physiol. 120:280-288; Jaffe, E.A., Ruggiero, J.T., Leung, L.K., Doyle, M.J., McKeown-Lango, P.J., Mosher, D.F. (1983). Proc. Nat. Acad. Sci. USA 80:998-1002). Bedingt durch die Kreuzreaktivitäten mit den eben erwähnten Normalgeweben sind falsch positive Signale beim Versuch, mit bekannten anti TSP-MAK in vivo Gefäßverschlüsse nachzuweisen, im allgemeinen zu beobachten.

Bei der Herstellung von αTSP-MAK gemäß Jaffe et al. (supra) haben wir überraschenderweise einen spezifischen MAK gegen aktivierte Plättchen (MAK BW 2128) gefunden, der im Westernblot nach Towbin T. & Gordon J. (1979) Proc. Natl. Acad. Sci. USA 76, 4350-4354 weder mit den bekannten a Granula Proteinen wie TSP, v. Willebrand Faktor oder gP 140 reagiert noch mit den oben genannten Zellen oder Geweben. Der MAK immunpräzipitiert ein Antigen, welches mit TSP assoziiert ist, einen innerhalb der experimentellen Fehlergrenzen von TSP nicht unterscheidbaren isoelektrischen Punkt besitzt, aber unter reduzierenden Bedingungen ein deutlich kleineres Molekulargewicht als TSP von ca. 160 kDa hat.

Die experimentellen Fehlergrenzen bei der Bestimmung des isoelektrischen Punktes betragen ca. ± 10-15 %, insbesondere ca. ± 5-10 %.

Gegenstand der Erfindung ist daher:
Ein Hybridom 2128 (DSM ACC2024).

Ein monoklonaler Antikörper, dadurch gekennzeichnet, daß er von dem Hybridom 2128 (DSM ACC2024) abstammt (BW 2128).

Monoklonale Antikörper oder Teile davon, die an ein Epitop, das von einem monoklonalen Antikörper des Hybridoms 2128 erkannt wird, binden.

Monoklonale Antikörper oder Teile davon, die präferentiell an aktivierte humane Plättchen binden und eine Aminosäuresequenz gemäß Tab. Ia und/oder Tab. Ib oder irgendeine allelische Variante oder Mutante davon, die die biologische Eigenschaft besitzt, an ein Epitop, das von dem monoklonalen Antikörper des Hybridoms 2128 erkannt wird, zu binden, enthalten.

Präferentielle Bindung bedeutet im Sinne der Erfindung, daß sich auf aktivierten Plättchen 3-1000 mal und insbesondere 10-100 mal mehr Epitope, die vom BW 2128 erkannt werden, befinden als auf nicht aktivierten Plättchen.

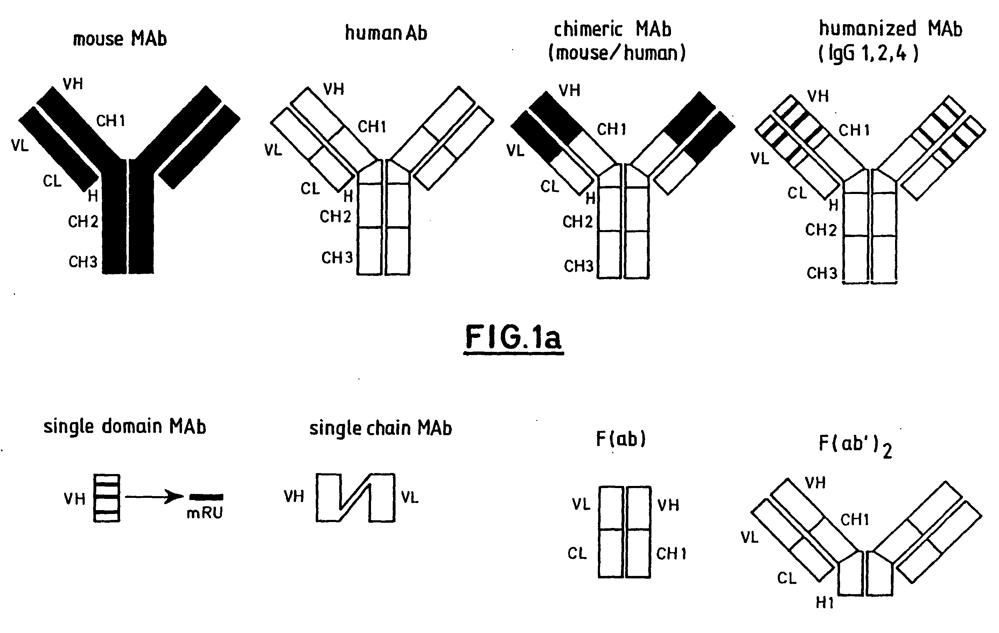

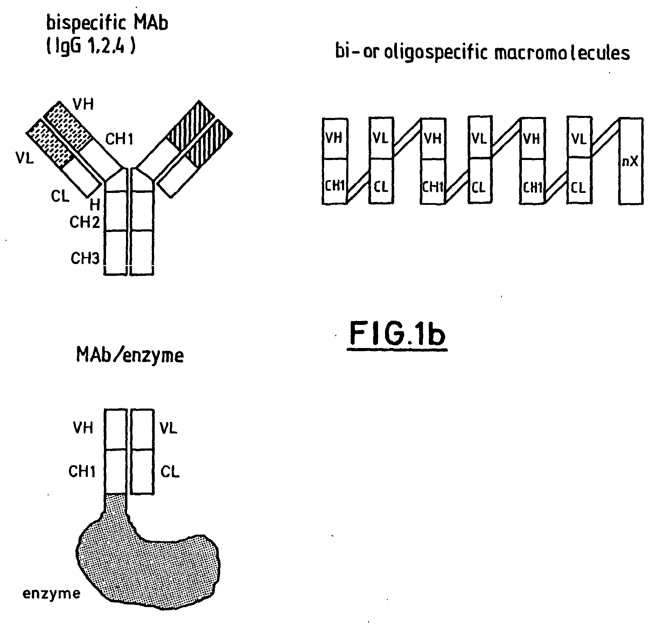

Ferner gehören zum Gegenstand der Erfindung monoklonale Antikörper oder Teile davon die chimärer, humanisierter, bi- oder oligospezifischer Art sind. Insbesondere fallen darunter ein mru-Fragment, ein single domain Fragment, ein single chain Fragment, ein F(ab)-Fragment oder ein F(ab')₂-Fragment mit einer oder mehreren Hinge-Regionen, wie beispielsweise in Figur 1a und 1b dargestellt.

Humanisierte Antikörper sind besonders bevorzugte Beispiele davon.

Ein mru (minimalrecognition unit) ist ein von einem CDR (Complementarity Determining Region) abgeleitetes Polypeptid, welches die Eigenschaft besitzt, an das vom spezifischen MAK erkannte Epitop zu binden.

Kleine organisch-chemische Verbindungen mit hohem Bindungspotential an das durch BW 2128 definierte Epitop kommen als geeignete Mimetika in Frage.

Im allgemeinen gehören zum Gegenstand der Erfindung Polypeptide, enthaltend eine Aminosäuresequenz gemäß Tab. Ia und/oder Tab. Ib oder irgendeine allelische Variante oder Mutante davon, die die biologische Eigenschaft besitzt, an ein Epitop, das von dem monoklonalen Antikörper BW 2128 erkannt wird, zu binden.

Besonders vorteilhaft ist es, wenn die erfindungsgemäßen monoklonalen Antikörper oder Teile davon oder die Mimetika als Fusionsproteine verwendet werden, die einen erfindungsgemäßen Immunglobulinanteil und einen nicht zur Immunglobulinfamilie gehörenden Teil enthalten.

Insbesondere soll das nicht zur Immunglobulinfamilie gehörende Protein ein fibrinolytisches Protein oder Teile davon sein, beispielsweise das fibrinolytische Protein Streptokinase, Urokinase, tPA, tPA-Mutanten oder Teile davon.

Ferner gehören dazu Vektoren, insbesondere die Expressionsvektoren L1 und L2 und geeignete Wirtszellen, insbesondere COS, CHO oder BHK Zellen, vorzugsweise BHK Zellen.

Ferner gehören zum Gegenstand der Erfindung Polynukleotide, die eine oder mehrere Nukleinsäuresequenzen enthalten, die für ein Polypeptid kodieren, das die biologische Eigenschaft besitzt, an ein Epitop, das von dem monoklonalen Antikörper BW 2128 erkannt wird, zu binden. Insbesondere fallen Nukleinsäuren gemäß Tab. Ia und Ib darunter, deren degenerierte Codons sowie Nukleinsäuren die gegen die Nukleinsäuren gemäß Tab. Ia und Ib unter allgemein bekannten Hybridisierungsbedingungen hybridisieren. Bevorzugte Hybridisierungsbedingungen sind stringente Bedingungen wie bei Sambrook, J., Fritsch, E.F., Maniatis, T. (1982), Molecular cloning: a laboratory manual, Cold Spring Harbor Laboratory Sambrook, J., Fritsch, E.F., Maniatis, T. (1989), Second Edition, Molecular cloning: A Laboratory Manual, Cold Spring Harbor Laboratory Press erläutert.

Ein weiterer Gegenstand der Erfindung ist ein Verfahren zur Herstellung eines erfindungsgemäßen Polypeptids, wobei eine prokaryotische oder eukaryotische Wirtszelle mit einem erfindungsgemäßen Polynukleotid transformiert oder transfiziert wird, so daß die Wirtszelle das genannte Polypeptid exprimiert. Das Polypeptid kann dann aus der Kultur nach dem Fachmann bekannten Methoden isoliert werden. Die allgemeinen gentechnischen Methoden sind dem Fachmann bekannt und können, wenn nicht anders, beispielsweise aus Molecular Cloning: A Laboratory Manual; Sambrook et al. (supra) entnommen werden.

Die erfindungsgemäßen Polypeptide können jedoch auch chemisch nach dem Fachmann bekannten Methoden der Peptidsynthese hergestellt werden.

Unter Polypeptid versteht man im Sinne der Erfindung Proteine oder Aminosäuresequenzen einschließlich ihrer räumlichen Konfiguration und posttranslationaler Modifikationen, wie Glykosylierung oder Phosphorylierung.

Im folgenden werden die Figuren kurz beschrieben:
**Fig. 1a:**
   Schematische Darstellung von Antikörpern, rekombinanten Antikörpern, Antikörperfragmenten und gentechnisch verknüpften Antikörperkonjugaten.
**Fig.1b:**
   Schematische Darstellung von Antikörperkonjugaten. Die verschiedenen Antigenbindungsspezifitäten des bispezifischen Antikörpers sind durch unterschiedliche Schraffierung der V-Bereiche verdeutlicht. Beim oligospezifischen Makromolekül sind jeweils die C_{H1} Domänen über Polypeptidlinker mit: den nächsten V_{H} Domänen verknüpft, \ während die leichten Ketten assoziiert sind. Beim Antikörper/Enzym Konjugat ist das Enzym gepunktet dargestellt.

Die Erfindung ist besonders geeignet in der in vivo Darstellung bzw. Therapie von Erkrankungen, bei denen aktivierte Plättchen beteiligt sind, wie z.B. Thrombosen, speziell beim Herzinfarkt oder Schlaganfällen, bei der thrombolytischen Therapie, tiefsitzenden Venenthrombosen, Lungenembolien, Gefäßverletzungen, atherosklerotischen Plaques, pyogenen Infektionen und Darmischämien.

Der MAK BW 2128 kann wie folgt hergestellt werden:

Humanes TSP wurde, wie von Dixit, V.M., Haverstick, D.M., O'Rourke, K.M., Hennessy, S.W., Grant, G.A., Santoro, S.A., Frazier, W.A. (1985), Biochemistry 24:4270-4275 beschrieben, aus aktivierten humanen Plättchen isoliert und zur Immunisierung von Balb/c Mäusen nach bekanntem Schema benutzt (Bosslet, K., Lüben, G., Schwarz, A., Hundt, E., Harthus, H.P., Seiler, F.R., Muhrer, C., Klöppel, G., Kayser, K., Sedlacek, H.H. (1985), Int. J. Cancer 36: 75-84). (Diese TSP Präparation kann mit anderen Plättchenproteinen kontaminiert sein.) Die Überstände der entstandenen Hybridome wurden auf kryopräservierten menschlichen Geweben mittels der indirekten APAAP-Technik (Cordell, J.L., Falini, B., Erber, W.N. et al. (1984), J. Histochem. Cytochem. 32:219) auf Spezifität untersucht. Im allgemeinen reagierten die Überstände mit aktivierten humanen Plättchen, dem Gangepithel der Leber, Gefäßen in der Niere, Lungenepithel, Milz und Knochenmark (Megakaryozyten). Sie zeigten also die literaturbekannte TSP-Spezifität. Es fand sich jedoch überraschenderweise auch ein Überstand, der spezifisch mit aktivierten humanen Plättchen reagierte aber nicht mit oben erwähnten Normalgeweben. Das Ergebnis war deshalb so überraschend, weil es in der zitierten Literatur keinen Hinweis gibt, der einen für aktivierte humane Plättchen spezifischen MAK erwarten ließ. Die Hybridomazelle, die diesen MAK sezernierte, wurde 3x mittels der "limited dilution" Technik kloniert und in flüssigen Stickstoff eingefroren (Hybridomazelle 2128). Eine detaillierte, immunhistochemische Spezifitätsanalyse ist in Tabelle II gezeigt. Über seine hohe Spezifität für Plättchen, die mittels der APAAP-Technik (Cordell et al. supra) nachgewiesen wurde, hinaus reagiert der MAK BW 2128 > 10x stärker mit Thrombin-aktivierten Plättchen als mit nicht aktivierten Plättchen. Formaldehydfixierung von Plättchen inhibiert die Bindung des MAK an Plättchen nur schwach. Zugabe des MAK zu nicht aktivierten humanen Plättchen führt zu keiner signifikanten Aktivierung, während die Zugabe von 0.5 Units Thrombin/ml eine starke Ausschüttung des Aktivierungsmarkers (PF4) bewirkt (Tabelle III). MAK, spezifisch für Epitope auf Granulozyten (z.B. BW 250) bzw. auf Mykoplasmen (z.B. BW 227) sowie gegen GPIa/IIa (z.B. BW4) haben ebenso wie der MAK BW 2128 keinen Einfluß auf die Aktivierung ruhender Plättchen (K. Bosslet et al. (1988) 14, 523-528). Die Methodik des ELISAs zur PF4-Messung nach Inkubation von Plättchen unter den in Tabelle III beschriebenen Bedingungen ist bei Pelzer, H., Heimburger, N. (1986), J. Biomed. Mat. Res. 20:1401-1409 sowie Osei-Bonsu, A., Cafourek, G., Reiter, S., Popovic, R., Sinzinger, H. (1987), Wiener klin. Wschr. 99:595-600 beschrieben. Diese Eigenschaften unterscheiden den MAK BW 2128 von den von Dixit, V.M., Haverstick, D.M., O'Rourke, K.M., Hennessy, S.W., Grant, G.A., Santoro, S.A., Frazier, W.A. (1985), Biochemistry 24:4270-4275 erzeugten anti TSP MAK sowie dem vcn Hayward, C.P.M., Warkentin, T.E., Horsewood, P., Kelton, J.G. (1991), Blood 77:2556-2560 beschriebenen anti Multimerin MAK JS-1, die die erwähnten biologischen Eigenschaften beeinflussen. Mittels Immunpräzipitation bzw. Western Blot konnte gezeigt werden, daß das von MAK BW 2128 erkannte Plättchenantigen nicht identisch ist mit gP 140, v. Willebrand Faktor, Fibronektin und Fibrinogen.

Mit Hilfe von MAK, die 3 unterschiedliche, nicht kreuzreaktive Epitope auf TSP erkennen (MAK BW 2125/8, BW 2126/332, BW 2126/662; das Epitopmapping wurde entsprechend der von Hammarström et al., Cancer Res. 49, 4852-4858, 1989, beschriebenen Methode durchgeführt), konnte gezeigt werden, daß obige MAK stark mit gereinigtem TSP reagieren, nicht aber an das mittels des MAK BW 2128 gereinigte Plättchenantigen binden. Diese Befunde wurden sowohl mittels eines Standard-Festphasen ELISAs als auch durch Western blotting-Analyse unter nicht reduzierenden Bedingungen erhoben. Diese Experimente zeigen, daß das durch den MAK BW 2128 definierte Plättchenantigen nicht die 3 Epitope trägt, welche von den oben erwähnten anti TSP MAK auf TSP erkannt werden, Zusätzlich war es möglich mittels 2D-Gelelektrophorese (O'Farrell, P.H. (1975), J. Biol. Chem. 250, 4007-4021) zu zeigen, daß der MAK ein Antigen präzipitiert, welches unter reduzierenden Bedingungen ein Molekulargewicht besitzt, welches kleiner ist als das von TSP und eine relative Molekularmasse von ungefähr 150.000 bis 170.000, vorzugsweise von ungefähr 160.000 besitzt, aber einen innerhalb der experimentellen Fehlergrenzen nicht unterscheidbaren isoelektrischen Punkt wie TSP besitzt, vorzugsweise im Bereich von ungefähr 5,5-6,5 (TSP hatte in einem Vergleichsexperiment ein Molekulargewicht von 180 KDa und einen isoelektrischen Punkt von 5,5 bis 6,5). Dieses neue Antigen ist mit TSP assoziiert, wobei sowohl eine physikalische wie auch eine chemische Bindung möglich ist und wird präferentiell auf aktivierten Plättchen exprimiert und daher im allgemeinen als Plättchenantigen bezeichnet. Das Antigen zeigt auch vorzugsweise die in Tabelle II aufgeführte Gewebeverteilung.

Das beispielsweise mittels Immunaffinitätschromatographie vorzugsweise mit Hilfe des MAK BW 2128 isolierbare Antigen eignet sich besonders gut für die Herstellung bzw. Überprüfung von zum MAK BW 2128 äquivalenten Antikörpern oder immunologisch reaktiven Teilen davon und für die Herstellung von Mimetika.

Die erfindungsgemäßen Antikörper lassen sich mit radioaktiven Isotopen markieren, insbesondere mit Tc-99m (Schwarz, A., Steinstraesser, A. (1987). J. Nucl. Med. 28:721) wenn sie vom IgG1 Isotyp sind wie der MAK BW 2128. Eine weitere Möglichkeit stellt die Markierung mit paramagnetischen Verbindungen dar. Die erfindungsgemäßen Antikörper und die Mimetika eignen sich demzufolge besonders gut für die Immunszintigraphische Darstellung von Gefäßverschlüssen.

Das Hybridom 2128 wurde bei der DSM, Deutsche Sammlung von Mikroorganismen und Zellkulturen GmbH (Mascheroder Weg 1B, 3300 Braunschweig) gemäß dem Budapester Vertrag unter der Hinterlegungsnummer DSM ACC 2024 hinterlegt. Des weiteren wurden die V-Gene der schweren und leichten Ketten des MAK BW 2128 nach der von Orlandi, R., Güssow, D., Jones, P.T., Winter, G. (1989). Proc. Nat. Acad. Sci. USA 86:3833-3837 beschriebenen Methode isoliert und nach der von Sanger, F., Nicklen, S., Coulson, A.R. (1977). Proc. Nat. Acad. Sci. USA 74:5463-5467 beschriebenen Methode die Nukleinsäuresequenz der essentiellen Bereiche des V-Gen Exons bestimmt (Tab. Ia, b).

Die klonierten V-Gene wurden als chimäre MAK BW Chi 2128 mit humanem verkürztem IgG3 Fc Teil (IgG3Δ) und humanem C-Kappa in BHK-Zellen ausgeprägt (Wirth, M., Bode, J., Zettlmeissl, G., Hauser, H. (1988). Gene 73: 419-426), um die Identität der klonierten V-Gene zu bestätigen (Beispiele A - N).

Die ausgeprägten BW Chi 2128 MAK zeigten die gleiche Antigenbindungsspezifität wie der Maus MAK. Des weiteren kann z.B. nach Polypeptidsynthese der CDRs bzw. von Teilen oder mehreren definierten CDRs die mrus (minimal recognition units) bestimmt und als wenig immunogene spezifische Peptide zur in vivo Lokalisation von aktivierten Plättchen eingesetzt werden. Des weiteren kann nach der von Saragovi, H.U., Fitzpatrick, D., Raktabutr, A., Nakanishi, H., Kahn, M., Greene, M.I. (1991). Science 253:792-795 beschriebenen Methode mittels organisch-chemischer Synthese ein Mimetikum mit hoher Spezifität und Avidität gegen das von MAK BW 2128 definierte Epitop erzeugt werden. Die humanisierte V-Region des MAK BW 2128 kann rekombinant z.B. mit Nukleotidsequenzen verknüpft werden, die für fibrinolytische Proteine wie Streptokinase, Urokinase, tPA, vor allem aber tPA-Mutanten kodieren. Bevorzugte tPA-Mutanten können beispielsweise nach EP-A1-0 387 380 hergestellt werden. Diese Konstrukte können wie in der deutschen Patentanmeldung P 41 06 389.9 (Ma 876) am Beispiel eines humanisierten αCEA MAK und der humanen *β*-Glucuronidase gezeigt, auf der Ebene der DNS verknüpft und als funktionelle Fusionsproteine exprimiert werden. Im allgemeinen stellen die genannten Fusionsproteine aufgrund der Kombination einer spezifischen hochaffinen Binderegion für aktivierte Plättchen in Gefäßverschlüssen und einer funktionell aktiven Region im gleichen Molekül effiziente und nebenwirkungsarme Fibrinolytika dar (Haber, E., Quertermous, Th., Matsueda, G.R., Runge, M.S., Bode, Ch. (1989). Jap. Circulation J. 54:345-353).

### Beispiel A:

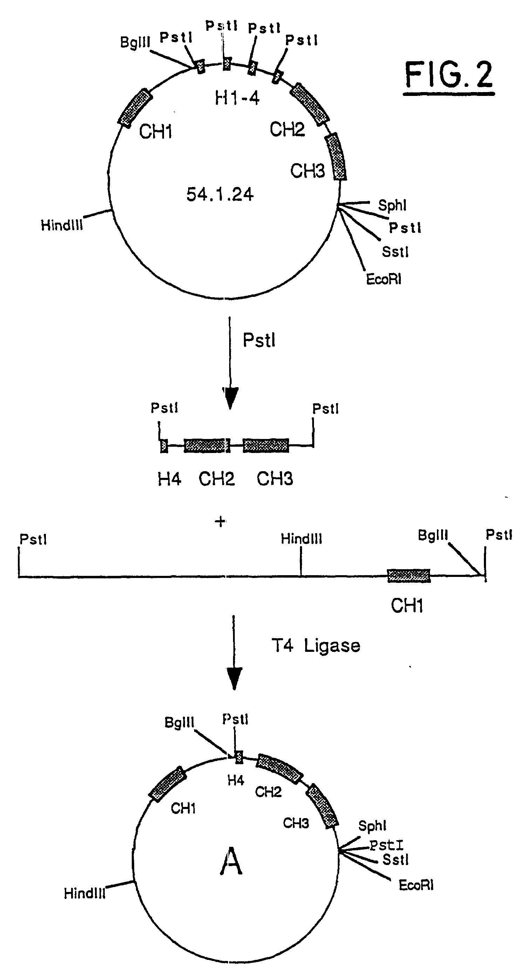

Der Plasmidklon 54.1.24, der das humane IgG₃ C-Gen trägt (DE 38 25 615 Al, Ma 706, Fig. 2) wurde mit PstI gespalten. Der dabei entstehende Vektor wurde mit dem größten der entstehenden Pstl Insert Fragmente ligiert und in Bakterien transformiert. Durch Restriktionsanalyse und Nukleinsäuresequenzbestimmung wurde der Plasmidklon A identifiziert, der ein humanes IgG₃ C-Gen trägt, bei welchem die H1, H2 und H3 Exons deletiert sind (Fig. 2).

### Beispiel B:

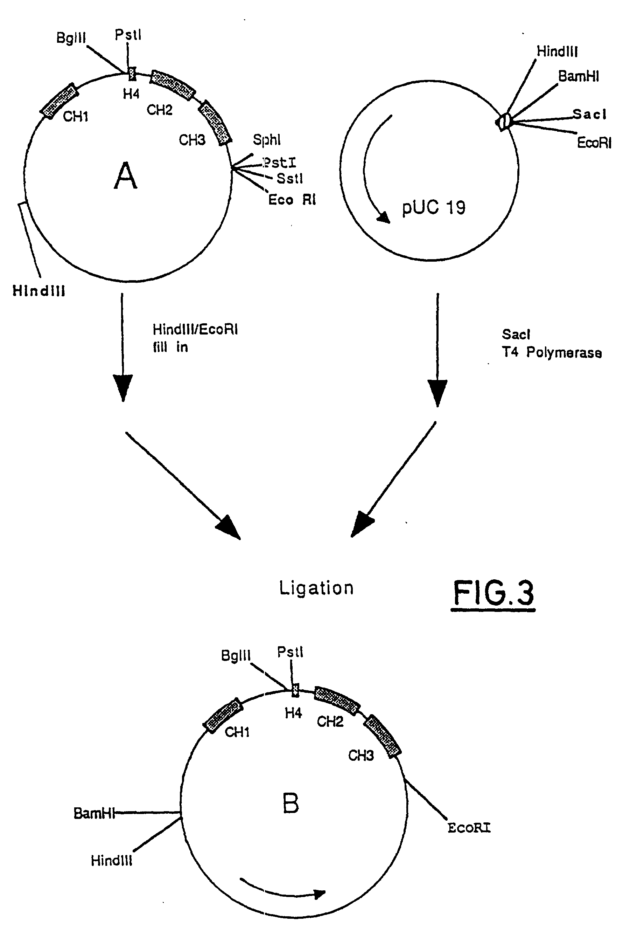

Der Plasmidklon A wurde mit HindIII und EcoRI gespalten, die Enden mit Klenow Polymerase aufgefüllt, das IgG₃ Insert isoliert und in einen mit SstI gespaltenen und mit Hilfe von T₄ Polymerase mit glatten Enden versehenen pUC19 Vektor ligiert. Durch Restriktionskartierung und Nukleinsäuresequenzanalyse wurde ein Plasmidklon B identifiziert, bei dem das IgG₃ Gen so orientiert ist, daß sich am 5' Ende die HindIII und am 3' Ende die EcoRI Schnittstelle des pUC19 Polylinkers befinden (Fig. 3).

### Beispiel C:

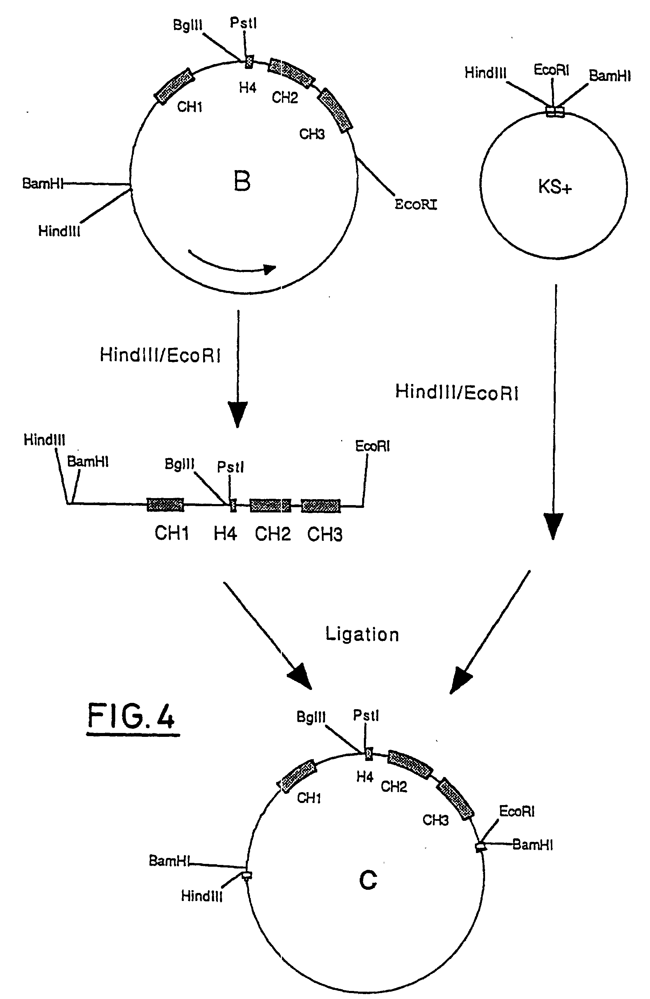

Das Plasmidklon B wurde mit EcoRI und HindIII gespalten, das IgG₃ Insert isoliert und in einen ebenfalls HindIII und EcoRI gespaltenen KS+ Phasmidvektor (pBluescriptII KS+; Stratagene, La Jolla, CA) ligiert. Es wurde der Phasmidklon C isoliert, bei dem das IgG₃ Gen am 5' und am 3' Ende von einer BamHI Schnittstelle flankiert wird (Fig. 4).

### Beispiel D:

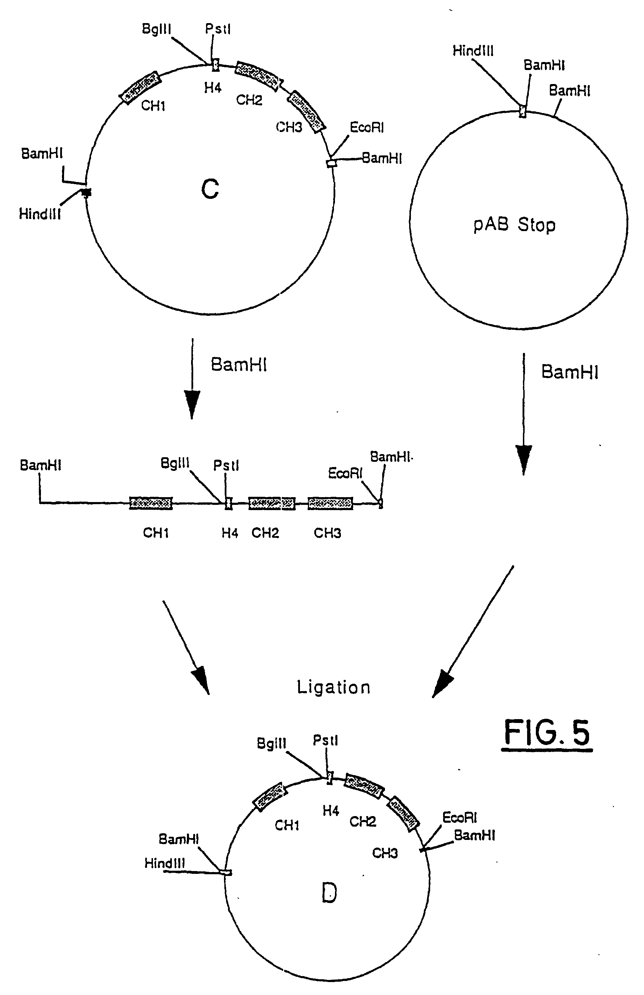

Der Phasmidklon C wurde mit BamHI gespalten, das IgG₃ Insert isoliert und in den ebenfalls mit BamHI gespaltenen Expressionsvektor pABStop (Wirth et al. supra) ligiert. Es wurde das Expressionsplasmid D identifiziert, welches das IgG₃ C-Gen in der in der Formel gezeigten Orientierung enthält. Bei dieser Klonierung verliert der pABStop Vektor das zwischen den beiden BamHI Schnittstellen gelegene SV40 Stop und Polyadenylierungssignal (Fig. 5).

### Beispiel E:

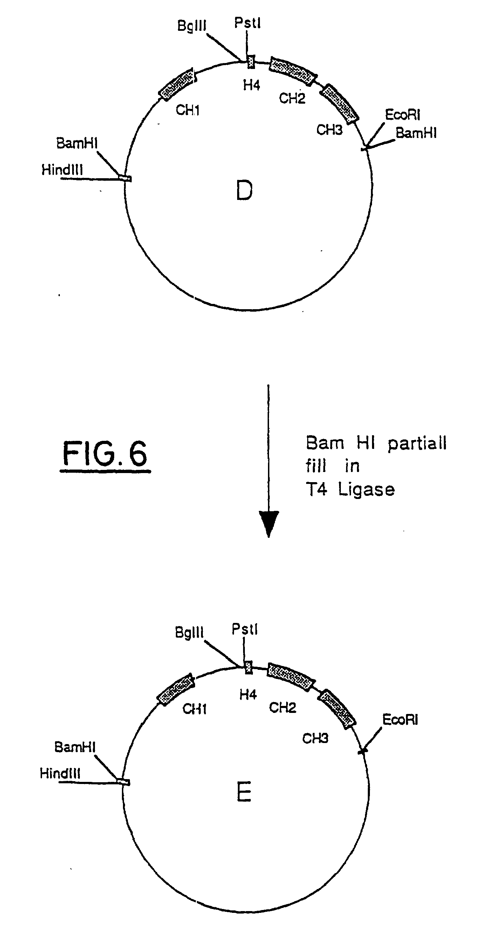

Das Expressionsplasmid D wurde mit BamHI partiell gespalten, die Enden mit Klenow Polymerase aufgefüllt und religiert. Es wurde das Expressionsplasmid E isoliert, bei dem die BamHI Schnittstelle 3' vom IgG₃ Gen zerstört ist (Fig. 6).

### Beispiel F:

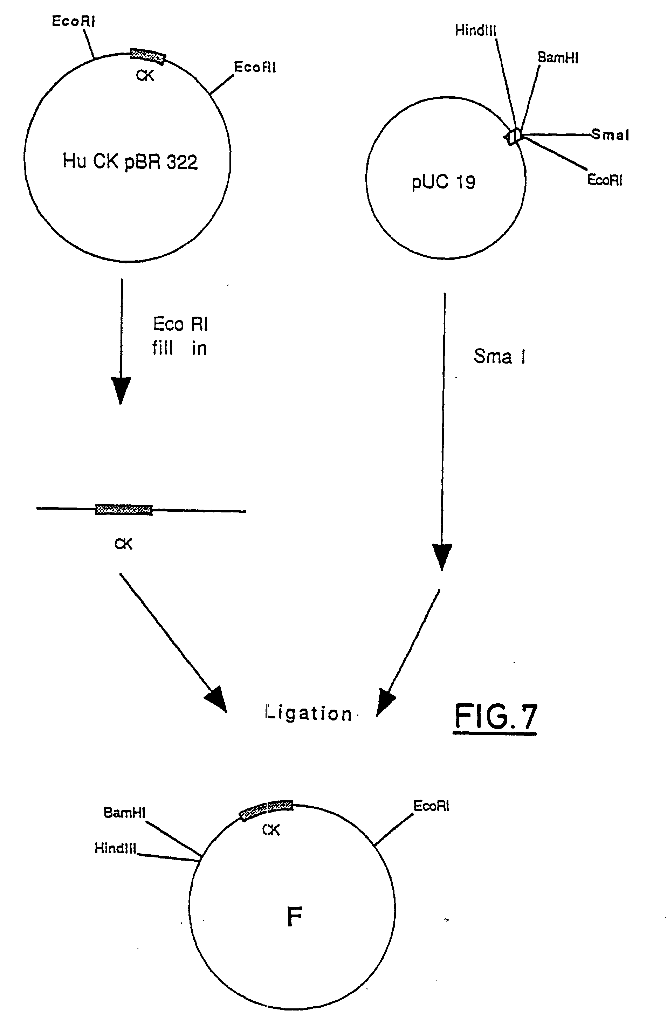

Das humane C-Kappa Gen (Hieter, P.A., Mainzel, J.V., Jr., Leder, P. (1982), The Journal of Biological Chemistry, 257:1516-1522) wurde als EcoRI Fragment in pBR 322 kloniert. Der pBR322 Vektor wurde mit EcoRI gespalten, die EcoRI Schnittstellen aufgefüllt, das C-Kappa Insert isoliert und in einen mit SmaI gespaltenen pUC19 Vektor ligiert. Es wurde der Plasmidklon F isoliert, bei dem das C-Kappa Gen am 5' Ende von einer HindIII und einer BamHI Schnittstelle und am 3' Ende von einer EcoRI Schnittstelle flankiert wird (Fig. 7).

### Beispiel G:

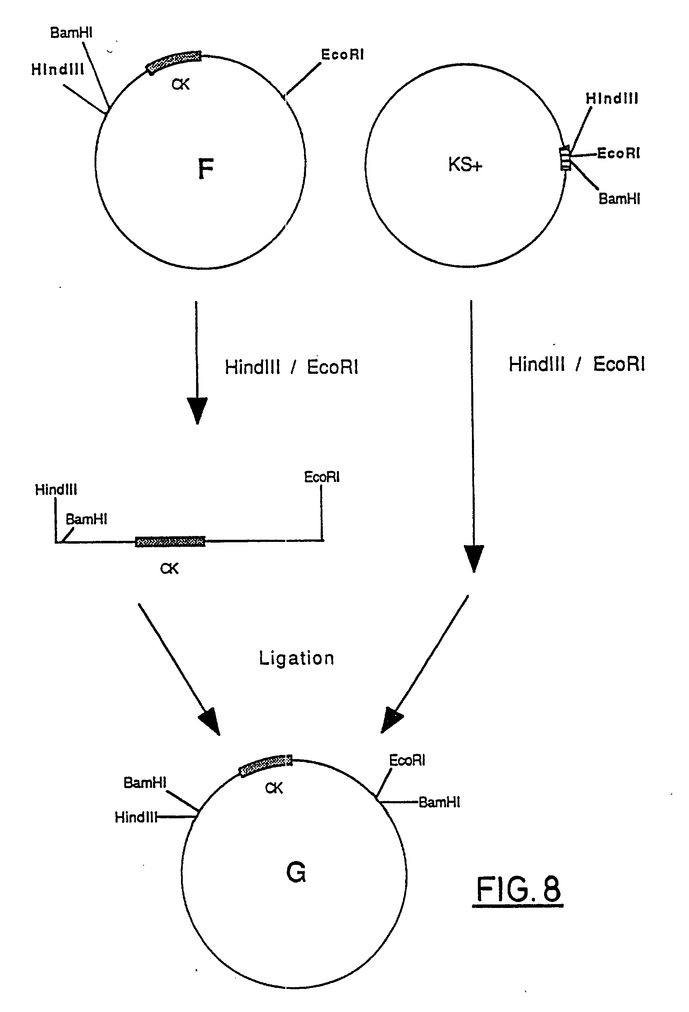

Der Plasmidklon F wurde mit HindIII und EcoRI gespalten, das C-Kappa Insert isoliert und in einen HindIII/EcoRI gespaltenen KS+ Phasmid kloniert. Es wurde der Phasmidklon G isoliert, bei dem das C-Kappa Insert am 5' und am 3' Ende von einer BamHI Schnittstelle flankiert wird (Fig. 8).

### Beispiel H:

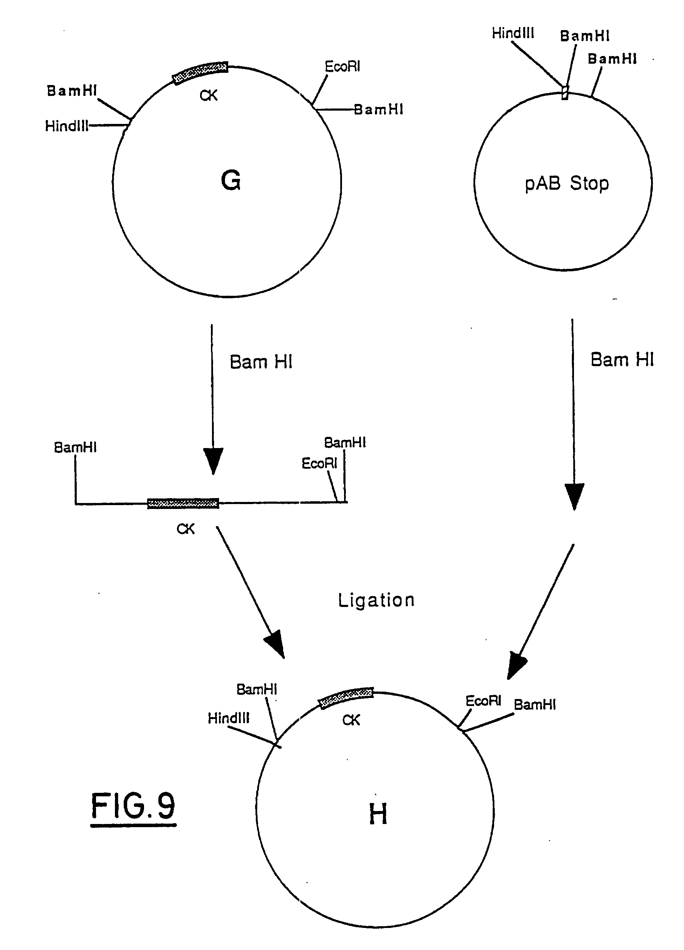

Der Phasmidklon G wurde mit BamHI gespalten, das C-Kappa Insert isoliert und in einen mit BamHI gespaltenen pAB Stop Vektor kloniert. Durch Restriktionskartierung und Nukleinsäuresequenzanalyse wurde der Klon H identifiziert, bei dem das C-Kappa Gen so orientiert ist, daß an seinem 5' Ende die HindIII Schnittstelle des pAB Stop Vektors liegt (Fig. 9).

### Beispiel I:

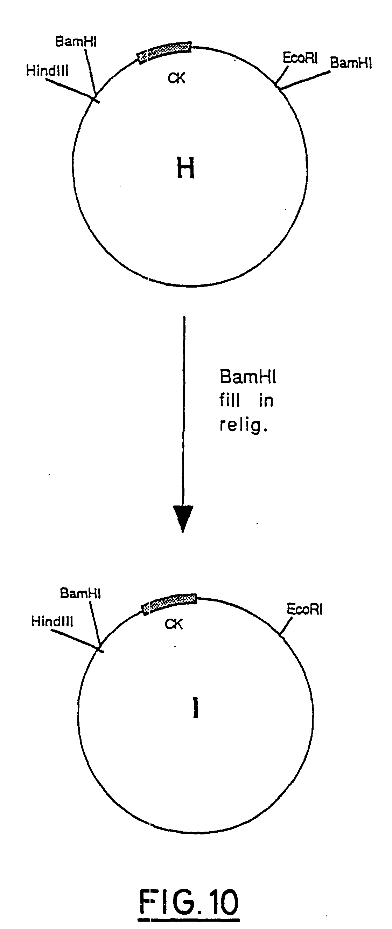

Der Klon H wurde mit BamHI partiell gespalten, die Restriktionsenden aufgefüllt und religiert. Durch Restriktionskartierung wurde der Klon I identifiziert, bei dem die BamHI Schnittstelle 3' des C-Kappa Gens zerstört ist (Fig. 10).

### Beispiel K:

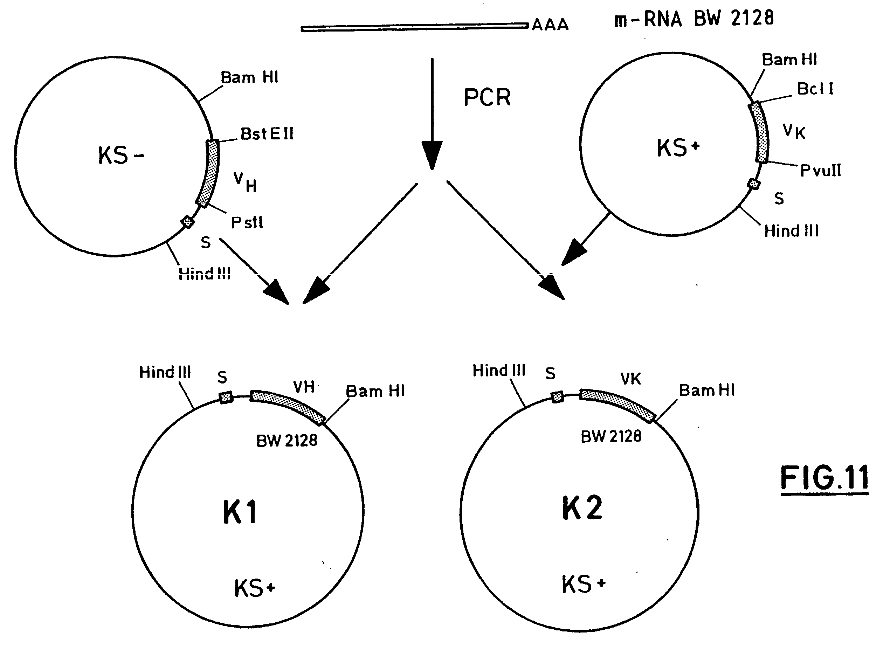

Die V_{H} und V_{K} Gene des MAK BW 2128 wurden nach Orlandi et al. (supra) mittels PCR Technik und spezifischer Oligonukleotide amplifiziert und in KS+ Vektoren kloniert (Güssow, D. und Seemann, G. (1992). Methods in Enzymology, Vol. 203, 99-121), welche unrelevante V_{H} und V_{K} Gene mit geeigneten Restriktionsschnittstellen enthielten. Es wurden anschließend die Klone K1 und K2 isoliert, bei denen die unrelevanten V_{H} und V_{L} Gene gegen die V_{H} (K1) und V_{K} (K2) Gene des MAK BW 2128 ausgetauscht sind (Fig. 11).

### Beispiel L:

Aus den Klonen K1 und K2 wurde die Nukleinsäuresequenz der V_{H} und V_{K} Gene des MAK BW 2128 nach der Methode von Sanger PNAS, 74, 5463, (1977) bestimmt (Tab. Ia, b).

### Beispiel M:

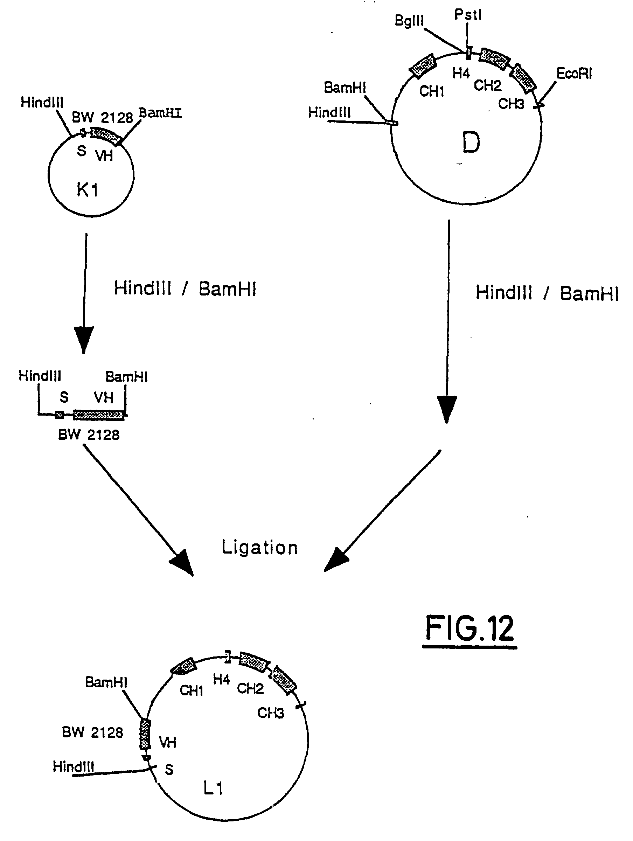

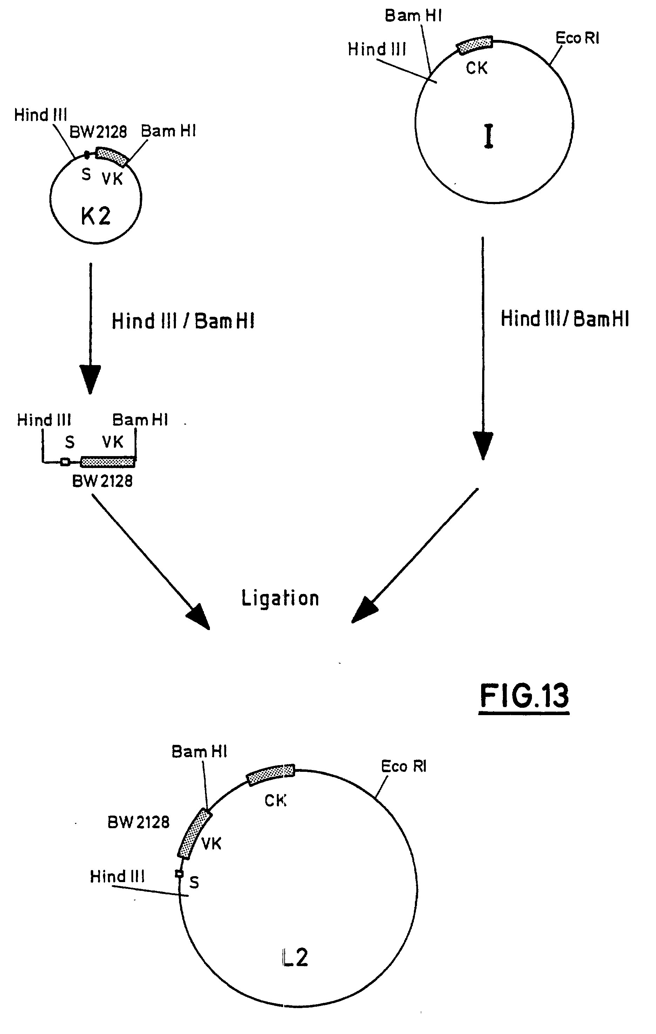

Aus den Klonen K1 und K2 wurden die V_{H} und V_{K} Gene des MAK BW 2128 mit Hilfe der Restriktionsenzyme HindIII und BamHI ausgeschnitten, die V-Gen Inserts isoliert und in die mit HindIII und BamHI geschnittenen Expressionsvektoren D(V_{H}) und I(V_{K}) kloniert. Es wurden die Expressionsvektoren L1 (Fig. 12) und L2 (Fig. 13) isoliert, die ein vollständiges Immunglobulin schwere Ketten Gen (L1) bzw. leichte Ketten Gen (L2) enthalten.

### Beispiel N:

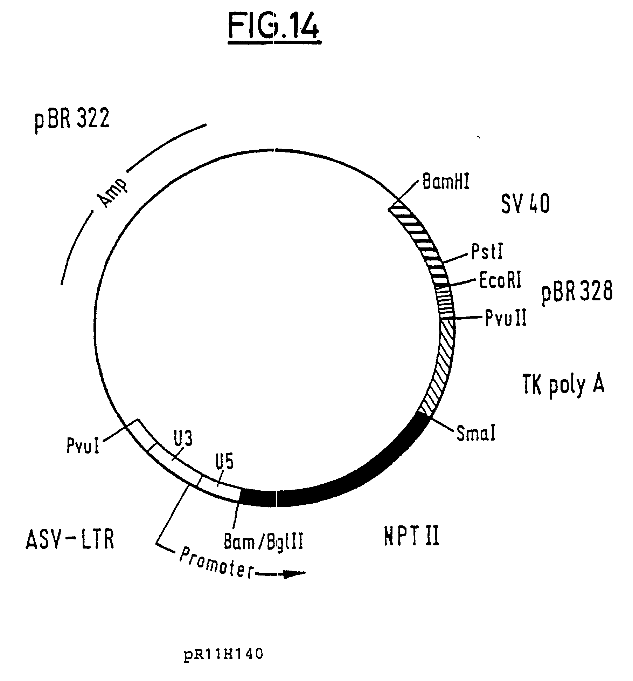

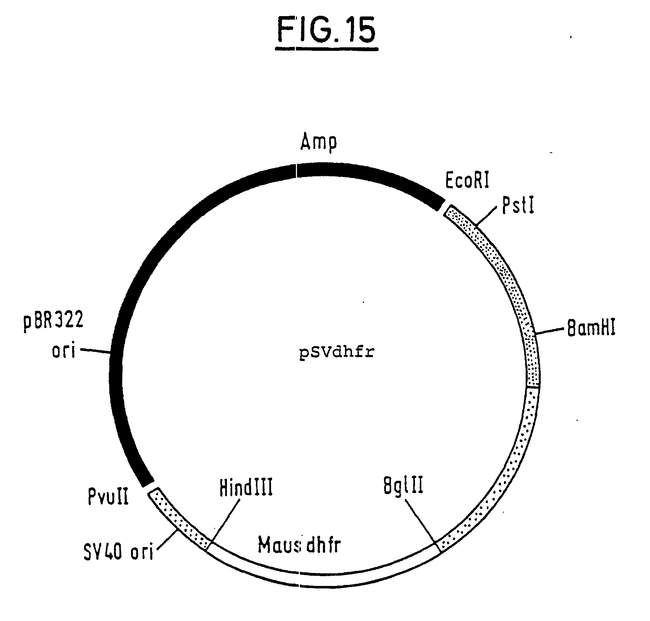

Die Expressionsvektoren L1 und L2 wurden zusammen mit dem Vektor pR11 H140 (Hudziak, R.M., Laski, F.A., RajBhandary, U.L., Sharp, P.A., Capecchi, M.R. (1982), Cell 31:137-146) der Neomycin Resistenz vermittelt (Fig. 14) und dem Vektor pSVdhfr (Lee, F., Mulligan, R., Berg, P., Ringold, G. (1981), Nature 294:228-232), welcher ein Dihydrofolatreduktasegen trägt und Resistenz gegen Methotrexat vermittelt (Fig. 15), in BHK-Zellen transfiziert (Zettlmeißl, G., Wirth, M., Hauser, H., Küpper, H.A. (1988). Behring Inst. Mitt. 82:26-34) und der ausgeprägte Antikörper auf Antigenbindungseigenschaften untersucht, um die Identität der V_{H} und V_{K} Gene des MAK BW 2128 zu bestätigen.

### Beispiel O:

Die immunhistochemische Spezifitätsanalyse der erfindungsgemäßen Antikörper erfolgte nach der APAAP-Technik (Cordell et al., supra). Tabelle II zeigt das Ergebnis einer immunhistochemischen Spezifitätsanalyse des MAK BW 2128.

### Beispiel P:

Die Plättchenaktivierung kann über eine quantitative Bestimmung des Aktivierungsmarkers PF4 mit Hilfe des bei H. Pelzer und N. Heimburger (supra) beschriebenen ELISAs gemessen werden. Tabelle III zeigt den Einfluß des MAK BW 2128 auf die Plättchenaktivierung.

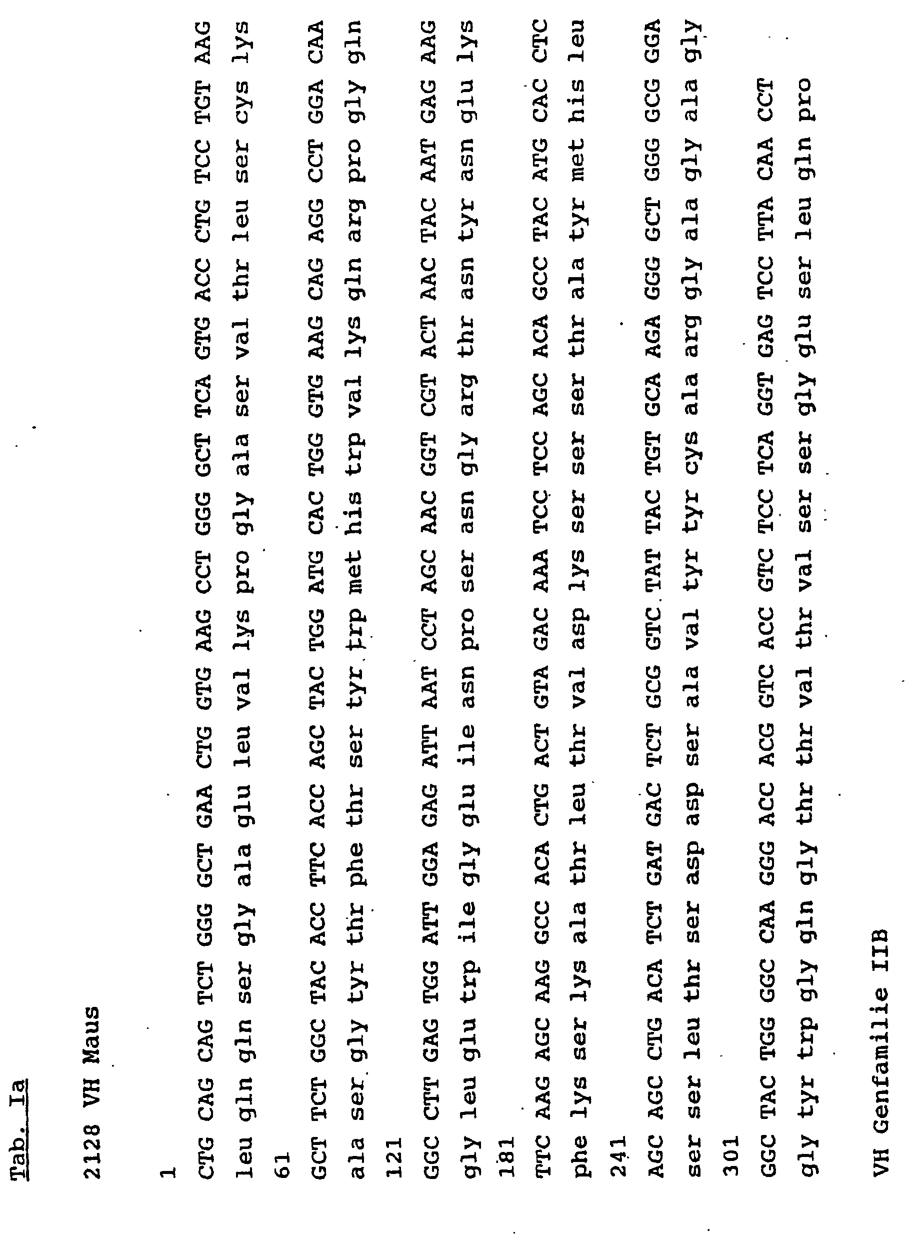

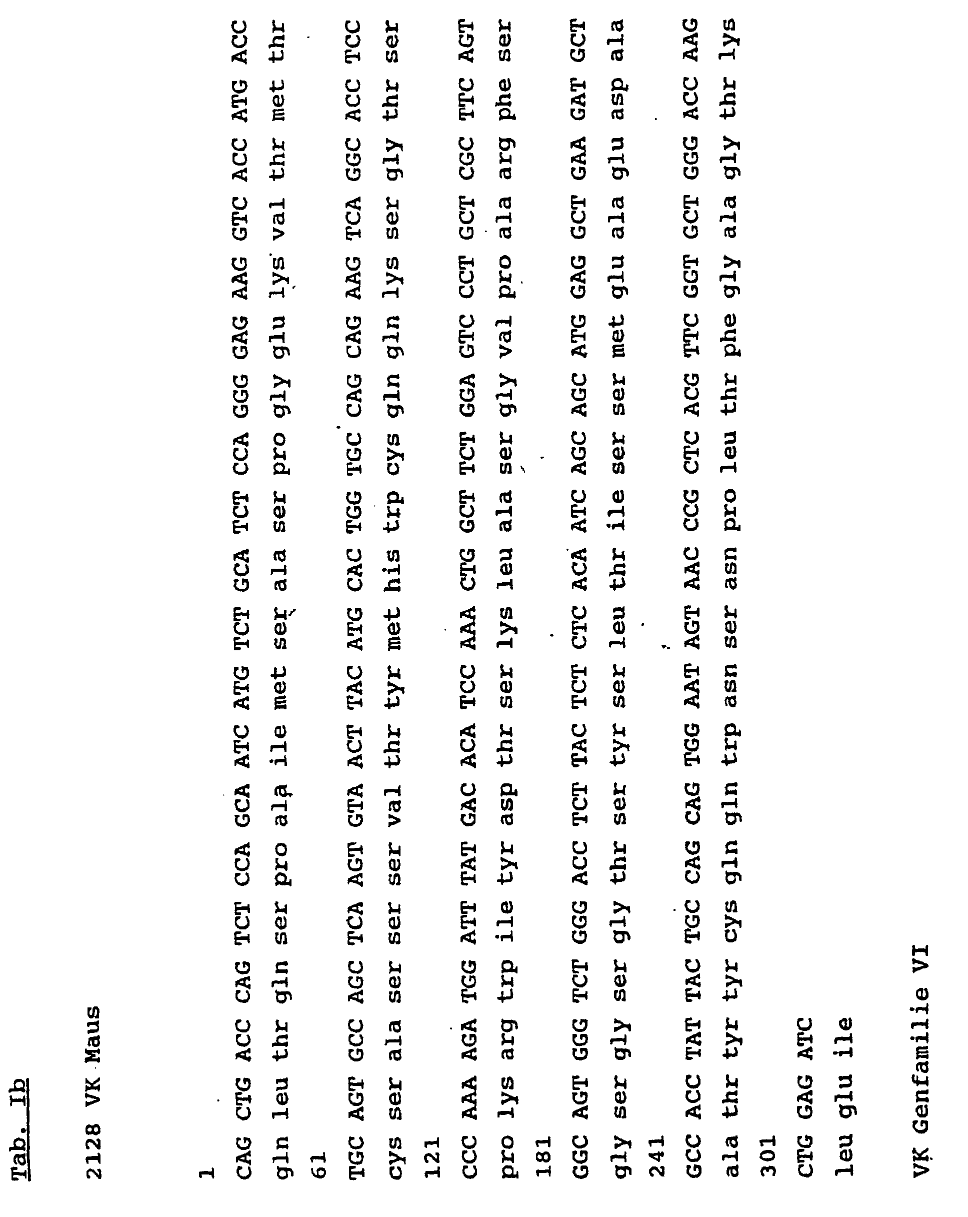

**Tab. II**

| Immunhistochemische Spezifitätsanalyse des MAK | | | |
|---|---|---|---|
| Gewebe | getestet | Reaktionstyp | |
| | | pos. | neg. |
| Thrombozytenpellet | 12 | +++ | |
| Granulozytenpellet | 10 | - | |
| Mono-/Lymphozytenpellet | 10 | +/++ | |
| Normalgewebe | | | |
| Lymphknoten | 2 | - | Bgfas - |
| Herz | 2 | - | Bgfas - |
| Herzbeutel | 4 | eThrom+/++ | Bgfas - |
| Nerven | 1 | - | Nervenfas - |
| Mandeln | t | - | Fas - |
| Milz | 4 | Throm++/+++ | Gef -, Fas - |
| Magenschleimhaut | 1 | - | Gef -, Fas - |
| Darmschleimhaut | 1 | ewThrom+ | Gef -, Fas - |
| Muskel | 5 | eSark, Fib+ | |
| Putamen | 1 | - | Fas - |
| Leber | 5 | eThrom++ | G -, Gef -, Fas - |
| Lunge | 4 | ewThrom+ | Gef -, Pneumo - |
| Knochenmark | 4 | ewThrom+ | Megakaryo. - |
| Niere | 3 | ewThrom+ | Gef -, Kan - |
| Humane Cas | | | |
| Magen Ca | 2 | ewThrom+ | Gef -, Bgfas - |
| Pankreas Ca | 2 | ewThrom+ | Gef - |
| Bronchial Ca- Adeno | 1 | - | Gef - |
| - Kleinz. | 1 | - | Gef - |
| - Platten. | 1 | - | Gef - |
| Mamma Ca | 2 | - | Gef - |
| Glioblastom | 2 | ewThrom+ | Gaf - |
| Melanom | 2 | ewThrom+ | Gef - |
| Colon Ca | 1 | - | Gef -, Bgfas - |
| Rectum Ca | 1 | - | Gef - |

Erklärung:
- ewThrom: - einige wenige Thrombozyten
- eThrom: - einige Thrombozyten
- G: - Gänge
- Gef: - Gefäße
- Kan: - Kanäle
- Bgfas: - Bindegewebsfasern
- Fas: - Fasern
- Pneumo: - Pneumozyten Typ 11
- Sark: - Sarkolemm
- Fib: - Fibrillen
- Megakaryo: - Megakaryozyten

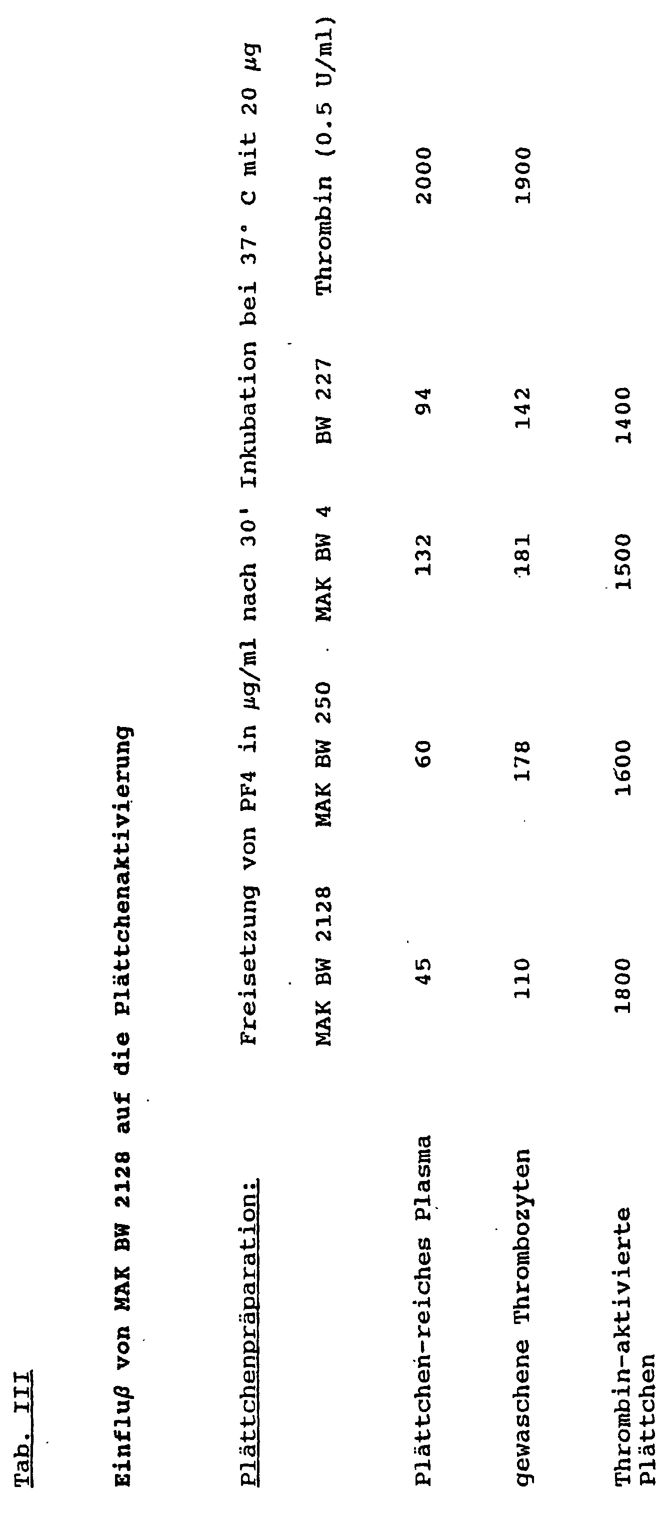

## Patentansprüche

1. Hybridom 2128 (DSM ACC2024)

2. Monoklonaler Antikörper mit Spezifität für aktivierte humane Plättchen, produziert von dem Hybridom 2128 (DSM ACC2024).

3. Monoklonale Antikörper oder Teile davon, **dadurch gekennzeichnet**, daß sie an ein Epitop, das von einem monoklonalen Antikörper nach Anspruch 2 erkannt wird, binden.

4. Monoklonaler Antikörper oder Teile davon nach Anspruch 2, **dadurch gekennzeichnet**,
daß der Antikörper ein chimärer, humanisierter, bi- oder oligospezifischer Antikörper ist.

5. Monoklonaler Antikörper nach mindestens einem der Ansprüche 2 -4, **dadurch gekennzeichnet**, daß er markiert ist, vorzugsweise mit Tc-99m.

6. Fusionsprotein, bestehend aus einem monoklonalen Antikörper oder Teilen davon, gemäß Anspruch 2 und einem nicht zur Immunglobulinfamilie gehörenden Protein oder funktionelle Teile davon.

7. Fusionsprotein nach Anspruch 6, **dadurch gekennzeichnet**, daß das nicht zur Immunglobulinfamilie gehörende Protein ein fibrinolytisches Protein oder funktionnelle Teile davon ist.

8. Fusionsprotein nach Anspruch 7, **dadurch gekennzeichnet**, daß das fibrinolytische Protein Streptokinase, Urokinase, tPA, tPA-Mutanten oder funktionelle Teile davon ist.

9. Pharmazeutische Zusammensetzung, enthaltend einen monoklonalen Antikörper nach mindestens einem der Ansprüche 2 - 5 oder Fusionsproteine nach mindestens einem der Ansprüche 6 bis 8.

10. Diagnostikum, enthaltend einen monoklonalen Antikörper nach mindetens einem der Ansprüche 2-5 oder Fusionsproteine gemäß mindestens einem der Ansprüche 6 bis 8.

11. Verwendung eiries monoklonalen Antikörpers nach mindestens einem der Ansprüche 2 -5 oder eines Fusionsproteins gemäß mindestens einem der Ansprüche 6 bis 8 zur Herstellung eines Diagnostikums zum Nachweis von aktivierten humanen Plättchen.

## Claims

1. The hybridoma 2128 (DSM ACC2024).

2. A monoclonal antibody having specificity for activated human platelets, produced by the hybridoma 2128 (DSM ACC2024) .

3. Monoclonal antibodies or parts thereof which bind to an epitope which is recognized by a monoclonal antibody as claimed in claim 2.

4. A monoclonal antibody or parts thereof as claimed in claim 2, wherein the antibody is a chimeric, humanized, bi- or oligospecific antibody.

5. A monoclonal antibody as claimed in at least one of claims 2 - 4, which is labeled, preferably with Tc-99m.

6. A fusion protein composed of a monoclonal antibody or parts thereof, as claimed in claim 2 and of a protein which does not belong to the immunoglobulin family, or functional parts thereof.

7. A fusion protein as claimed in claim 6, wherein the protein which does not belong to the immunoglobulin family is a fibrinolytic protein or functional parts thereof.

8. A fusion protein as claimed in claim 7, wherein the fibrinolytic protein is streptokinase, urokinase, tPA, tPA mutants or functional parts thereof,.

9. A pharmaceutical composition containing a monoclonal antibody as claimed in at least one of claims 2 - 5, or fusion proteins as claimed in at least one of claims 6 to 8.

10. A diagnostic aid containing a monoclonal antibody as claimed in at. least one of claims 2 - 5, or fusion proteins as claimed in at least one of claims 6 to 8.

11. The use of a monoclonal antibody as claimed in at least one of claims 2 - 5, fusion or a protein as claimed in at least one of claims 6 to 8 for the preparation of a diagnostic aid for detecting activated human platelets.

## Revendications

1. Hybridome 2128 (DSM ACC2024).

2. Anticorps monoclonal à spécificité pour les plaquettes humaines activées, produit par l'hybridome 2128 (DSM ACC2024)

3. Anticorps monoclonal ou parties de celui-ci, **caractérisés en ce qu** 'ils se lient à un épitope qui est reconnu par un anticorps monoclonal selon la revendication 2.

4. Anticorps monoclonal ou parties de celui-ci selon la revendication 2, **caractérisés en ce que** l'anticorps est un anticorps chimère, humanisé, bi- ou oligospécifique.

5. Anticorps monoclonal selon au moins une des revendications 2 à 4, **caractérisé en ce qu**'il est marqué, de préférence par Tc-99m.

6. Protéine de fusion, constituée d'un anticorps monoclonal ou de parties de celui-ci selon la revendication 2 et d'une protéine n'appartenant pas à la famille des immunoglobulines ou de parties fonctionnelles de celle-ci.

7. Protéine de fusion selon la revendication 6, **caractérisée en ce que** la protéine n'appartenant pas à la famille des immunoglobulines est une protéine fibrinolytique ou des parties fonctionnelles de celle-ci.

8. Protéine de fusion selon la revendication 7 **caractérisée en ce que** la protéine fibrinolytique est la streptokinase, l'urokinase, le tPA (activateur tissulaire du plasminogène), des mutants de tPA ou des parties fonctionnelles de ceux-ci.

9. Composition pharmaceutique contenant un anticorps monoclonal selon au moins une des revendications 2 à 5 ou des protéines de fusion selon au moins une des revendications 6 à 8.

10. Agent de diagnostic contenant un anticorps monoclonal selon au moins une des revendications 2 à 5 ou des protéines de fusion selon au moins une des revendications 6 à 8.

11. Utilisation d'un anticorps monoclonal selon au moins une des revendications 2 à 5 ou d'une protéine de fusion selon au moins une des revendications 6 à 8, pour la préparation d'un agent de diagnostic destiné à la détection de plaquettes humaines activées
